# EUROPEAN PATENT SPECIFICATION

(11) **EP 2 623 587 B1**
(45) Date of publication and mention of the grant of the patent: **20.11.2019**
(21) Application number: 12153868.0
(22) Date of filing: 03.02.2012
(51) Int. Cl.: C12M 1/34, C12M 1/36, C12M 1/00

(54) **Apparatus and method for automated replacement of culture medium and performing toxicity assays on live cells**
Vorrichtung und Verfahren zum automatisierten Austausch von Kulturmedium und Durchführung von Toxizitätstests an lebenden Zellen
Appareil et procédé de remplacement automatisé de milieu de culture et de réalisation d'analyses de toxicité sur des cellules vivantes

(43) Date of publication of application: 07.08.2013
(73) Proprietor: Cellix Limited, 8 Dublin (IE)
(72) Inventor: Shvets, Igor, Dublin, D15 (IE); Kashanin, Dmitry, Dublin, D18 (IE); Dohen, Christophe, Dublin, D8 (IE); Paul, Toby, Dublin, D24 (IE)
(74) Representative: Purdylucey Intellectual Property

(56) References cited:
- WO-A1-2005/113742
- JP-A- 2004 089 138
- US-A- 5 565 353
- US-A1- 2006 281 169
- DATABASE WPI Week 200264 Thomson Scientific, London, GB; AN 2002-593861 XP002677347, -& JP 2002 153256 A (CHIYODA CORP) 28 May 2002 (2002-05-28)

## Description

### Technical Field

This invention relates to enabling the replacement of the culture medium for live cells, including mammalian derived cells. It provides the method and apparatus for delivery of liquids such as cell culture liquids and other reagent containing liquids. The delivery of such liquids can be used to carry out experiments and tests that can include biological experiments, experiments for medical research, drug development, diagnostic tests and other such similar experiments. The invention can also be used for cell production, tissue culture and protein drug production. The invention can be used with manual-controlled or programmed pumps to feed cells placed in conventional laboratory tissue culture flasks, Petri dishes and multi-Petri dishes plates. The invention can also be used with WIFI technologies for control of the pump. The system can be applied in the field of bioreactors, where it is necessary to separate incoming fresh medium and the outgoing media containing by-products of biological cells growth.

### Background of the Invention

In modern cell biology, biotech, drug development or medical laboratory small-scale cell culture is performed using a variety of specialized containers such as cell culture flasks, Petri dishes or multiwell plates. These containers come in different volume sizes and with different surface treatments optimized for specific types of cells and particular experiments. Usually a cap or a lid of the tissue culture container is gas permeable or it is not air-tight sealed against the container's body. This ensures exchange of air between the interior of the container and the ambient when the container is placed inside culture incubator. Such exchange of air is important for supporting appropriate conditions for cell life and growth, maintaining the desired pH in the culture medium release of gas originating from the biological processes within the media. Furthermore, cell incubators are often equipped with gas controllers for mixing of nitrogen, oxygen and carbon dioxide gases at predetermined ratio to support cell culture.

In addition to delivering gas to the growing cells, the culture media contains nutrients and salts necessary for the cell growth and division. It may also contain other liquids necessary for biological experiments. As the cells undergo their life cycle, the medium becomes starved of nutrients and also filled with cell by-products, which need to be disposed. The cell growth rate is largely dependent on the cell type. Some cells are slow growing and their doubling time is high: Hep-G2 Liver cancer (48hrs), MIA PaCa-2 Epithelial carcinoma (48hrs), Mesenchymal Stem cells (45-68hrs). Some cells are moderate growing: MCF-7 Breast cancer (29hrs), LNCaP Epithelial carcinoma (35hrs), MDA-MB-231 (30hrs) ; and fast growing: Human Embryonic stem cells (12-18hrs).

Depending on the cell type in the culture, the medium inside each flask (cell container) may need to be exchanged for a fresh culture medium typically every 10-14 hours. This routine of laboratory cell culture is carried out inside a tissue culture laminar flow hood to ensure aseptic conditions and it is often manual and laborious.

Some cell types require continuous supply of fresh medium, and disposal of a used medium to ensure their physiological growth. Such cells types are liver cell (hepatocytes) and embryonic stem cells. The growth of these cells is difficult to sustain when manual culture techniques are applied. Successful cultivation of different cell types demands not only skillful handling, but also depends to a large extent on the chosen cultivation system. Continuous perfusion technology closely mimics the physiological conditions within the body enabling production of drastically increased cell densities and more organotypic cell morphologies, sometimes even to the extent of achieving 3-dimensional cell growth.

Recent advances related to automation of cell culture can be divided into two distinct groups.

First group of inventions is focused on improvements to tissue culture vessels to aid automatic withdrawal of culture medium.

US patent 6908767B2 to Bader et al (2005) describes method for growing cells in an automated manner for diagnostic purposes. A plate having plurality of holes or wells is used to culture cells. Unlike commonly used multiwell plate, the bottom of the plate described in the invention, is gas permeable to allow transfer of oxygen to the cultured cells. Additionally, the individual wells need to be sealed in order to inject and withdraw the culture medium. The invention describes ways by which the well can be sealed and also method to ensure that the culture medium delivers nutrients to the cells uniformly. The drawback is that the exact amount and proportion of carbon dioxide and oxygen delivered to cells is unknown. It depends on several parameters such as permeability of the film, flow rate of delivery of gas and also on amount of medium and the rate of flow of medium in the well. Control of oxygen to carbon dioxide ratio can be important in order to support sustain desired PH of the medium, one of the key parameters for cell culture. Additionally the patent describes the use of membrane bottom of a well to deliver the gas to the cells. It is unclear how the well can be sealed, as pressure during perfusion can eventually transfer the medium outside the well through the porous membrane.

US patent 7749750B2 to Kobayashi et al (2010) describes a culturing apparatus having two distinct parts: a culture vessel having the elastic seal bonded to it with two ports for injection and withdrawal of culture medium and the second part having microinjection means capable of connecting to the delivery system. The two parts are sealed via the elastic seals, which provide a convenient method for robotic automation, which is also described in the invention. Additionally the system introduces seals allowing access to the culture vessel by robotic dispensers and at the same time ensuring sterile operation. It is unclear form the invention how the necessary conditions of the cell culture are supported: temperature, PH and gas constitution of the culture medium.

US patent 2007/0031963 A1 to Chang et al. (2007) describes cell culture flask modified for various automated processes such as introduction and removal of culture medium. The access to the cell culture vessel body is allowed via specially designed closures, which can accommodate blunt tips and close after withdrawal of the tip from the cell culture vessel body. The closures are pre-pierced to allow easy access with blunt pipette tips. These advanced cell culture vessels are used without the lid, which eliminate the need for using robotic arms to automating cell culture.

US patent 7816128 B2 to Nakashima et al. (2010) does not deal with modifications to cell culture vessels, but provides method of automated cell culture using robotic apparatus capable of conveying culture vessels. The system consists of at least one incubator capable of accommodation culture vessel, the dispensing and suction machine and robot for opening lids of culture containers such as Petri dishes.

Second group of inventions is focused on the design of integrated systems containing purpose built cartridge, bioreactors or perfusion chambers to host the cells. These systems are usually fully enclosed and include perfusion pump, system of valves and gas control apparatus.

US patent 7270996B2 to Cannon et al (2007) provides the bio-culture platform including closed-loop flowpath cartridges for growing cells, system for sampling and collection of culture medium capable of integration with analysis system for evaluating the status of growing cells. The cartridges used for growing cells are connected via series of ports to a perfusion path capable of circulating the culture medium and also maintaining required oxygen and carbon dioxide concentration via oxygenator units. The medium can be delivered through the closed-loop flowpath once or repetitively. The medium reservoir is provided as the source of culture medium connected via oxygenator to the bioreactor cartridge output of which is connected to the waste reservoir. There are embodiments with valves introduced into the flow path to divert culture medium for PH or other analysis sampling. Although, the invention provides an integrated culture system, it requires purpose built cartridges and multiplicity of auxiliary components to operate. Additionally an oxygenator module would only operate successfully if medium flows through it continuously; otherwise there will be a difference in medium oxygenation inside the oxygenator and in the cartridge where cells grow. This may lead to consumption of large amount of fresh medium if the system is used in the single pass flow mode.

Another integrated bio-culture system is described in US patent 2011/0212493A1 to Hirschel et. al. (2011). The invention includes construction of bioreactor integrated with the cell culture system. Similar to the previously described patent the flow path includes auxiliary components to ensure the correct pH and oxygen concentration of medium. A particular feature of this patent is that the design of the bioreactor perfusion chamber allows for growth of significant amount of cells due to the large surface area provided for the growth. This is achieved by spiraling the interior surface of the bioreactor and also by pleating the said surface.

WO2005/113742 describes a device for regulation of the filling of a culture vessel with culture medium that employs a closed cell culture vessel connected to a fresh cell culture medium vessel and a used cell culture medium container, and in which the fresh cell culture medium vessel and used cell culture medium container are open to ambient.

Despite all the efforts in developing automated cell culture systems, majority of routine cell culture is still done manually. In practice, the main obstacle in implementing automated cell culture system is cost associated either with equipment or consumables. Recent technological advances in this area usually do not require costly or elaborate flasks or cell culture vessels but they do require complicated robotic system to automate operation of the cell culture apparatus and enable replacement of the culture medium. In the case of integrated systems and bioreactors, the cell culture containers are purpose built, often limited to culture of a particular cell type and are costly.

### Statements of Invention

The present invention provides a novel system and method for laboratory-scale cell culture compatible with conventional tissue culture containers (flasks) available on the market and routinely used in cell biology laboratories or in medical laboratories. The system and method for laboratory-scale cell culture is further compatible with conventional cell culture incubators and can be used for a variety of adherent and non-adherent cell types.

The invention further typically provides a system with a low dead volume and capable of handling low volumes of cell culture media, comparable to the ones used in static cell culture experiments.

The present invention further typically provides system and method for laboratory-scale automated cell culture without use of robotic arms, dispensers or other complicated robotic machinery. This minimizes the cost of cell culture instrumentation and routine and allows the system to be applied in small- and medium scale laboratories.

All the media-carrying components of the system, excluding the pump, such as tubing, bottle and cap components can generally be disinfected by autoclaving and therefore can be reused. The pump can be disinfected by washing with appropriate disinfectant fluid and cell culture flask, which is disposable.

The system allows both for introduction of fresh culture medium and removal of the culture medium containing by-products of cell life-cycle using a single pump, without use of valves.

The system allows continuous perfusion of fresh culture medium and withdrawal of the culture medium containing by-products of cell life-cycle, which allows growing cell under more physiological conditions.

The invention provides cell culture system for laboratory-scale bioreactor applications, where outgoing consumed culture medium does not come in contact with the incoming fresh culture medium and outgoing media contains proteins, enzymes, hormones and antibodies that need to be collected for disposal or for further analysis.

The system allows for remote programming of the perfusion time and number of perfusion cycles per day using Smartphone connected to the pump controller via Wi-Fi network.

Broadly, the invention provides an apparatus suitable for enabling replacement of the culture medium that live cells are grown in according to Claim 1.

The cell culture vessel is not sealed to ambient (i.e. it is not airtight), and the cell culture storage and collection vessels are generally pressurized during use. This is generally achieved by providing a pressure equalization conduit between the storage and collection vessels such that the pressure exerted on one of the vessels by the pump is transferred to the other vessel. In this embodiment, the storage and collection vessels need to be sealed to the outside. Generally, the ends of the pressure equalization conduit are located in the headspace of the respective vessels, although in one embodiment one or both of the ends may be located within culture medium.

In one embodiment, the fresh culture medium storage vessel and used culture medium collection vessel are formed by separate compartments of the one container.

The invention also relates to a method of perfusing cells in a cell culture vessel with fresh cell culture medium, which method employs an apparatus according to the invention and comprises the steps of:
- adding fresh culture medium to the fresh culture medium storage vessel such that the inlet of the supply conduit is immersed within the culture medium;
- adding fresh culture medium to the cell culture vessel such that the inlet of the drainage conduit is immersed within the culture medium; and
- actuating the pump to pump fresh culture medium from the storage vessel to one end of the cell culture vessel and withdraw used cell culture medium from the second end of the cell culture vessel.

The cell culture vessel is typically a cell culture flask or a Petri dish, although the use of other cell culture vessels is envisaged.

In one embodiment, the pump is provided in-line in the supply or drainage conduit, ideally the supply conduit.

The pump is typically a solenoid pump or a peristaltic pump, although the use of other types of pumps is envisaged. The pump is adapted to be actuated periodically, for example once every 5 mins, 10 mins, 30 mins, hour, 2 hours, 3 hours, 4 hours, 6 hours, 12 hours, 24 hours. The pump may also be adapted to be remotely operated by means wireless communications, for example over a WI-FI network.

Suitably, the fresh culture medium is a liquid suitable for culturing live cells.

In another embodiment, the fresh culture medium contains a drug substance and the apparatus is used for monitoring cell status over time.

In another embodiment, the fresh culture medium contains substances required for promoting cell growth and division.

In another embodiment, the fresh culture medium contains a substance capable of inducing cell differentiation between different cell types.

In another embodiment, the fresh culture medium also contains a substance capable of inducing cells to produce cytokines, proteins, antibodies or other cell life cycle by-products.

The invention also provides a method of performing a toxicity assay on live cells, which method employs a method of perfusing live cells with cell culture medium according to the invention, wherein the cell culture medium includes a test agent, and wherein the method includes a step of assaying the toxicity of the test agent to the cells in the cell culture vessel.

Typically, the test agent is selected from a toxin or a drug, although other test agents may be employed for example food additives, agents found in industrial effluents, bacterial or viral components, and the like.

### References cited:

6908767 B2 to Bader et al (2005),
7749750 B2 to Kobayashi et al (2010),
2007/0031963 A1 to Chang et al. (2007),
7816128 B2 to Nakashima et al. (2010),
7270996 B2 to Cannon et al (2007),
2011/0212493 A1 to Hirschel et al. (2011).

### Brief Description of Drawings

The invention will be more clearly understood from the following description of some embodiments thereof given by way of example only with reference to the accompanying figures in which:
Figure 1 Overview of the apparatus.
Figure 2 Schematics of the apparatus showing cross-sections of the cell culture vessel, fresh culture medium storage vessel, used culture medium collection vessel, the pump and the conduits.
Figure 3 Automated cell culture system view with culture medium bottle having two compartments.
Figure 4 Integration of automated cell culture system with cell culture incubator and Smartphone.
Figure 5a Image of MDA-MD-231 cell line in the T75 cell culture flask 2 hours after seeding
Figure 5b Image of MDA-MD-231 cell line in the T75 cell culture flask after 96 hours of perfusion
Figure 6a Image of WM793 cell line in the T75 cell culture flask 2 hours after seeding
Figure 6b Image of WM793 cell line in the T75 cell culture flask after 130 hours of perfusion

### Detailed Description of the Invention

The invention can be best understood from the description of the following drawings showing a number of embodiments. The embodiments given do not form an exhaustive list but rather are examples.

The term "culture medium" as used throughout this document should be understood in a broad sense as being any liquid, generally an biological liquid, including liquids for supporting cell growth or liquid for cell assays or liquid containing drugs. Thus, for example, the apparatus is suitable for performing toxicity studies on live cells where the culture medium being provided to the cells includes a test agent, for example a putative toxin or a drug.

The term "monitoring cell status" should be understood to mean monitoring cell growth, cell death, apoptosis, cell activation, cell morphology, cell motility, and the general or specific production by the cell of molecules such as proteins, sugars, hormones, antibodies, and the like.

The term "cells" may be eukaryotic or prokaryotic cells. Generally, they are mammalian cells, usually human cells, although the apparatus and methods of the invention may be employed for enabling the replacement of culture medium in other types of cells, for example bacterial or viral cells.

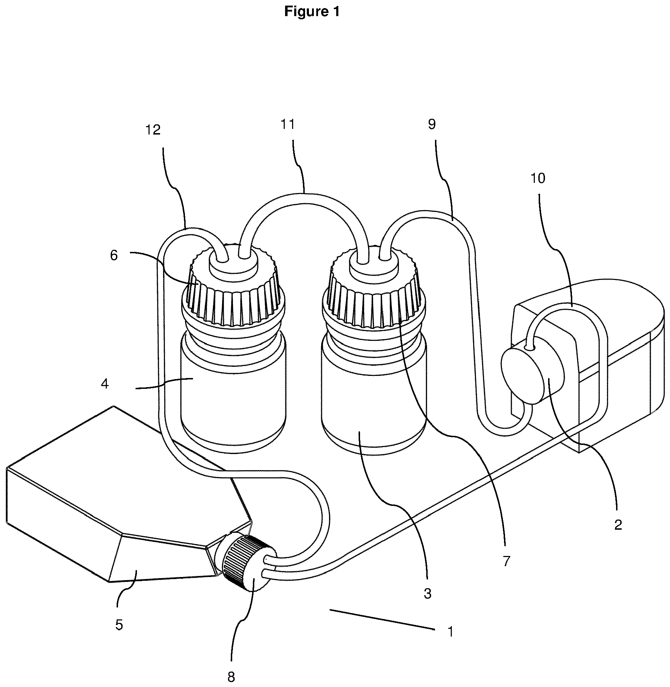

Figure 1 shows the apparatus for cell culture indicated by a numeral 1. The apparatus comprises the pump 2, the fresh culture medium storage vessel 3, the used culture medium collection vessel 4, and the cell culture vessel 5. The fresh culture medium storage vessel 3 is a bottle closed with the bottle cap 7 in the way that no gas or liquid can escape or enter the bottle, except through the ports in the cap. One way to do it to use threaded bottles and threaded caps with Teflon insert seal. The used culture medium collection vessel 4 is a bottle closed with the bottle cap 6 in similar way to the bottle cap 7. The cell culture vessel 5 is a flask capped with cell culture vessel cap 8, but not air tight so that gas can exchange between the interior of the flask and the ambient. The cap of the cell culture vessel is threaded and the thread not tightened fully. Alternatively, the cap (8) can be devised with a gas permeable membrane (not shown in Fig. 1). The fresh culture medium storage vessel 3 is connected to the pump 2 via pump inlet conduit 9 and also to the used culture medium collection vessel 4 via pressure transfer conduit 11. In a typical embodiment the conduits 9 and 11, as well as the conduits 10 and 12 could be made of flexible polymer tubing of round or another cross-section. The pump 2 is connected to the cell culture vessel 5 by via pump outlet conduit 10. The cell culture vessel 5 is also connected to the used culture medium collection vessel 4 by culture medium collection conduit 12.

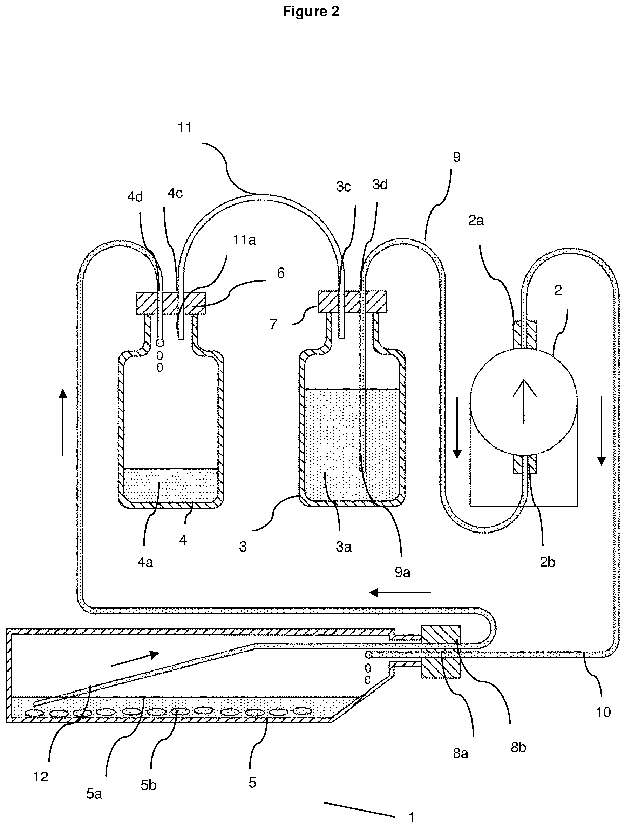

Figure 2 shows schematics of cross-section of cell culture system 1 and in particular the transfer path of fresh and used culture medium. The pump inlet conduit 9 is connected to the bottle cap 7 via pump inlet tube port 3d. The pressure transfer conduit 11 is also connected to the bottle cap 7 via corresponding transfer port 3c. The pump inlet tube port 3d and transfer port 3c ensure that the connection is airtight so that the interior of the culture medium vessel cannot exchange gas with the ambient. There are numerous configurations of such an airtight port. In one example it is a cylindrical hole with a compression ring made out of rubber as commonly known in industry. In this case both, pump inlet conduit 9 and pressure transfer conduit 11 are hard-wall plastic tubes, for example made out of PTFE polymer. Other examples of air tight connections between the bottle cap 7 and the conduits 9 and 11 will be known to those skilled in the art. In a similar way culture medium collection conduit 12 is connected to the collection tube port 4d, pressure transfer conduit 11 is connected to the transfer port 4c.

It is not necessary to seal the distal end of the pump outlet conduit 10 against the inlet port 8a of the cell culture vessel in airtight manner and likewise the culture medium collection conduit 12 does not need to be sealed in air tight manner against flask outlet port 8b of the cell culture vessel.

Before commencing the cell culture experiment, the fresh culture medium storage vessel 3, the used culture medium collection vessel 4, cell culture vessel cap 8, bottle cap 6 and bottle cap 7 and all the tubing are autoclaved to ensure sterility. Alternatively, these can be taken from sterile packs if disposable such components are being used. The pump 2 is washed with the disinfectant solution, for example 70 % ethanol, and then flushed with air to remove excess disinfectant. The culture medium storage vessel 3 is filled with fresh culture medium 3a preferably with the neck of the vessel being left empty. The bottle cap 7 is applied to the fresh culture medium vessel 3 in air tight manner. The conduits 11 and 9 are connected according to the description above so that the opening 9a of the pump inlet conduit 9 is immersed into the fresh culture medium 3a and the opening 11a of the pressure transfer conduit 11 is not. The culture medium collection vessel 4 is empty or near to empty. The cell culture vessel 5 is filled with live cells 5b and the cells are allowed to settle and attach to the bottom of the cell culture vessel 5 . It will be appreciated by those skilled in the art that the cells may need to be placed on scaffold or matrix and reagents may need to be added to stimulate the cell experiments, or cell growth or cell assay. Details of the specific arrangements depend on the type of assay to be carried out.

During the experiment, the pump 2 starts operating and withdraws a preset volume of fresh culture medium from the culture medium vessel 3 and transfers it to the cell culture vessel 5. The pump outlet conduit 10 delivers fresh culture medium to the cells. The pump outlet conduit 10 can be in contact with cell culture medium 5a or be placed out of direct contact, e.g. above surface of the cell culture medium 5a. The latter case can be advantageous for bioreactor applications. As the pump 2 withdraws the volume of medium from the fresh culture medium vessel 3 it creates reduction in pressure of the gas directly above the fresh medium culture medium 3a. This reduction in pressure is transferred to the used culture medium collection vessel 4 via pressure transfer conduit 11. As culture medium collection conduit 12 is immersed in the cell culture medium 5a, the reduction in pressure inside the culture medium collection vessel 4 results in the flow of medium from the cell culture vessel 5 to the used culture medium collection vessel 4. The direction of the flow is indicated by arrows in Figure 2. As the system is fully enclosed, the volume of medium added to the cell culture vessel 5 via the pump outlet conduit 10 and the volume withdrawn from the cell culture vessel 5 by the reduction in pressure via that culture medium collection conduit 12. The flow of the medium stops after the pressure in the culture medium vessel 3 returns to the initial pressure. It takes another stroke of the pump 2 to repeat this cycle. Repeated strokes of the pump result in continuous transfer of the culture medium.

The used culture medium 4a has no direct contact with cell culture medium 5a. This allows the apparatus 1 to ensure that the waste products or by -products of cell life cycle are not affecting cell growth.

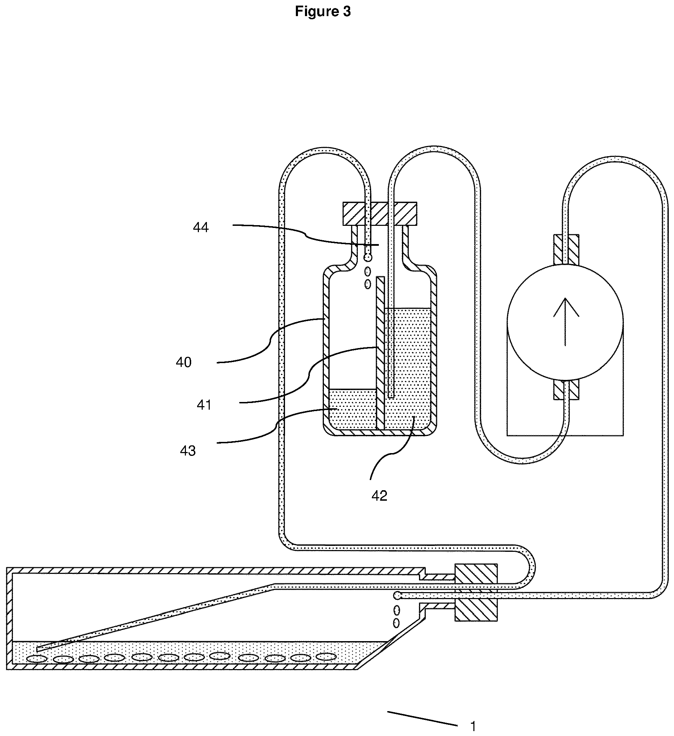

Figure 3 displays another embodiment of the cell culture system 1 where only one culture medium vessel is used. It is essential that the interior of the culture medium vessel is separated by the divider. The culture medium dual vessel 40 is in this configuration capable of holding both fresh culture medium 42 and also used culture medium 43. The transfer of pressure between two compartments of the bottle is achieved via the bottle neck 44. The conduits are connected to the bottle cap as described above in the airtight manner.

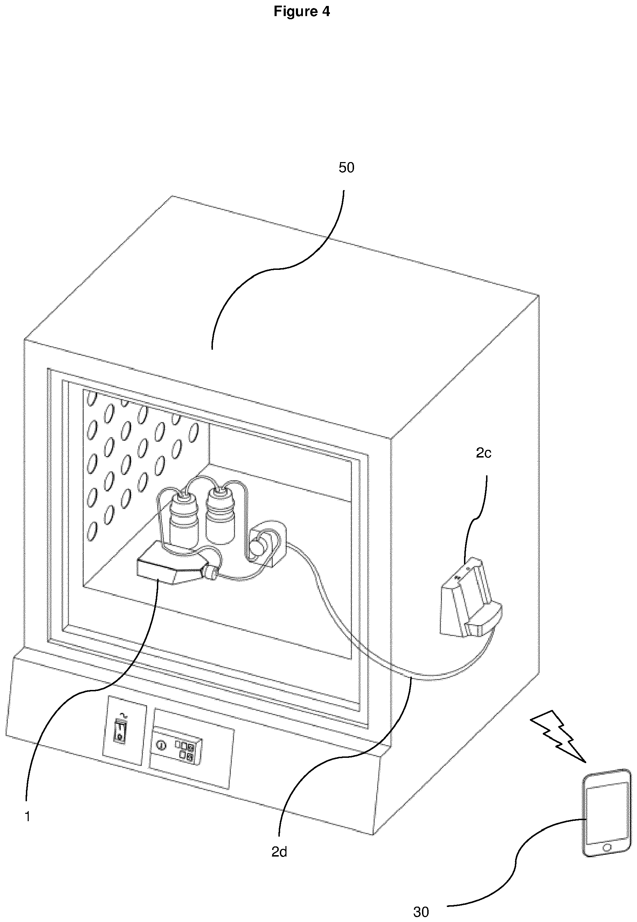

Figure 4 shows a view of apparatus 1 integration with cell culture incubator and Smartphone. For the purpose of better presentation the door of the incubator is omitted from the drawing. The apparatus 1 can be placed on the shelf of the cell culture incubator 50. The pump 2 is connected to the pump controller 2c by the pump drive cable 2d. This cable supplies power to the pump drive and also transmits signal proportional to the flow of the pump back to the pump controller 2c. The pump controller 2c is powered of the main supply and controlled by the remote controller. In the embodiment shown the remote controller is smartphone 30. The pump controller 2c can be preprogrammed by smartphone 30 prior to the experiment. For example users can select perfusion period, perfusion flow rate and the time between consecutive perfusions by using specific smartphone software application. The communication between the pump controller 2c and the smartphone 30 can be remote via WI-FI or Bluetooth network. Additionally, the pump controller 2c can be manually programmed via keypad.

The system described above is capable of maintaining culture of various cell types. Following are two example of adherent cell culture using MDA-MD-231 breast cancer cell line and WM793 skin cancer cell line.

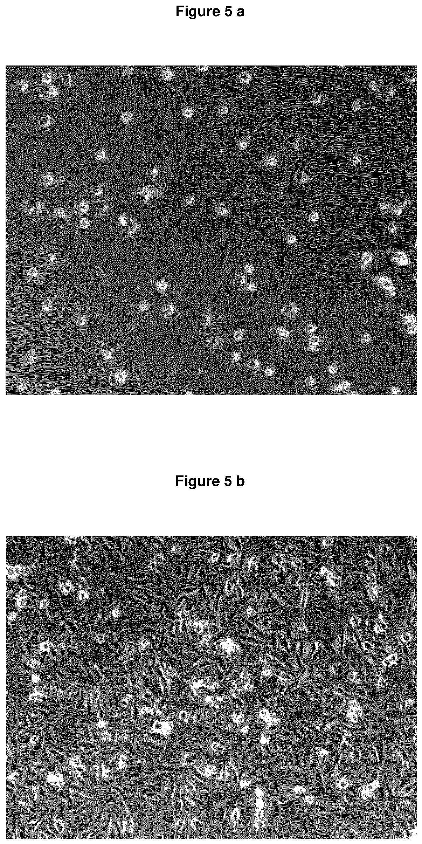

Prior to the experiment MDA-MD-231 cells were first grown in growth medium (RPMI 1640, FBS-10%, 2mM L-glutamine, 100µg/ml Penicillin/Streptomycin). The cells were trypsinized using Trypsin-EDTA (0.05%) solution and resuspended in 20ml of medium in T-75 cm² flask. Flask was transferred to CO₂ incubator and incubated for two hours for the cells to attach to the surface. Figure 5a shows the image of cells inside the flask prior to perfusion. This image was taken with inverted microscope equipped with phase contrast and digital camera. After incubation flask was connected to described perfusion system inside the Biosafety hood and the whole system and flask has been transferred inside cell culture incubator. The media was refreshed every 2 hours with pump running for 10min under defined flow rate of 100 ul/min. The experiment had run for 96 hours. Fig 5b shows the image of cells inside the flask after 96 hours of perfusion.

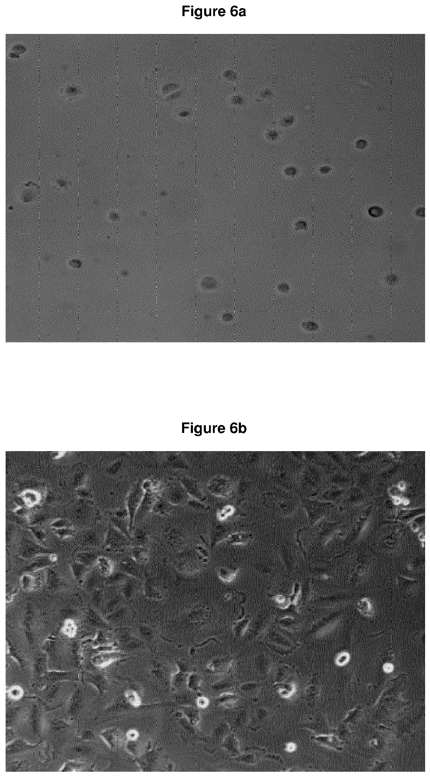

Similar technique was used for culture WM793 skin cancer cell line. Prior to the experiment cells were first grown in growth media (RPMI 1640, FBS-10%, 2mM L-glutamine, 100µg/ml Penicillin/Streptomycin). The cells were trypsinized using Trypsin-EDTA (0.05%) solution and resuspended in 20ml of media in T-75 cm² flask. Flask was transferred to CO₂ incubator and incubated for two hours for the cells to attach to the surface. Figure 6a shows the image of cells inside the flask prior to perfusion. This image was taken with inverted microscope equipped with phase contrast and digital camera. After incubation flask was connected to described perfusion system inside the Biosafety hood and the whole system and flask has been transferred inside cell culture incubator. The media was refreshed every 2 hours with pump running for 10min under defined flow rate of 100 ul/min. The experiment had run for 130 hours. Fig 6b shows the image of cells inside the flask after 96 hours of perfusion.

The described system can also be used for production of antibodies, cytokines, enzymes and hormones. Below, we give two examples of such applications for monoclonal antibody and virus production:
Monoclonal antibodies are antibodies with a defined specificity derived from cloned cells or organisms. They can be obtained from immortalised B-lymphocytes that are cloned and expanded as continuous cell lines (murine and human monoclonal antibodies) or from rDNA-engineered mammalian or bacterial cell lines (engineered monoclonal antibodies). The monoclonal antibodies are produced by Hybridoma cellines, which have to be cultured for several days to get the maximum and optimal production of desired antibody. A wide range of anti-mouse and anti-human monoclonal antibody producing hybridoma cell lines are available. For example Hybridoma celline 60H9(9)D10.E6 (Derived from a patient with chronic Hepatitis C) is cultured using media RPMI 1640 + 2mM Glutamine + 1% Non-Essential Amino Acids (NEAA) + 1% Sodium Pyruvate (NaP) + 20u/ml IL-6 + 10% Foetal Bovine Serum (FBS). The product obtained after continuous culture for 10-15 days is Immunoglobulin G (IgG1) (kappa), which is specific for Hepatitis C virus NS4 region.

Numerous research applications, including small-scale virus production, rely on T-flasks for cell culture. One example is the production of Adenovirus. HEK293 cells are grown to 80% confluence in T75 culture flasks. GFP adenovirus is added at 100:1 multiplicity of infection and the cells have to be perfused for 3 to 4 days for optimal production of viruses. Similarly Vero cells (African GreenMonkey, adult kidney, epithelial) are used to produce polioviruses.

## Claims

1. An apparatus (1) for enabling the replacement of culture medium for live cells:
- a cell culture vessel (5);
- a fresh culture medium storage vessel (3);
- a used culture medium collection vessel (4);
- a supply conduit (9, 10) adapted to provide fluid communication between the fresh culture medium storage vessel (3) and the cell culture vessel (5);
- a drainage conduit (12) adapted to provide fluid communication between the cell culture vessel (5) and the used culture medium collection vessel (4), an inlet of the drainage conduit being disposed within the cell culture vessel (5) such that in use it is it located in the culture medium; and
- a pump (2) adapted to pump fresh culture medium from the fresh culture medium storage vessel (3) to the cell culture vessel (5) and to pump used culture medium from the cell culture vessel (5) to the used culture medium collection vessel (4),
wherein:
the cell culture vessel (5) is not sealed to ambient, and the apparatus includes a pressure equalization conduit (11) adapted to provide pressure equalization between the used culture medium collection vessel (4) and the fresh culture medium storage vessel (3) and in which the fresh culture medium storage vessel (3) and used culture medium collection vessel (4) are sealed to ambient

2. An apparatus as claimed in Claim 1 in which the fresh culture medium storage vessel (3) and the used culture medium collection vessel (4) are sealed so that they cannot communicate with the ambient, with the only communication to and from the said vessels (3) and (4) being via the supply conduit (9, 10), drainage conduit (12), and equalization conduit (10).

3. An apparatus as claimed in Claim 1 or 2 in which the pump (2) is provided in-line in the supply or drainage conduit (9, 10, 12).

4. An apparatus as claimed in any of Claims 1 to 3 in which the fresh culture medium storage vessel (3) and the used culture medium collection vessel (4) are formed by separate compartments of the one container (40).

5. An apparatus as claimed in any of Claims 1 to 4 in which the cell culture vessel is selected from a Petri dish and a cell culture flask.

6. An apparatus as claimed in any preceding Claim in which the pump is adapted for remote operation by means of wireless communication system.

7. An apparatus as claimed in any preceding Claim in which the cell culture vessel (5) is not sealed to the ambient.

8. A method of perfusing cells in a cell culture vessel with fresh cell culture medium, which method employs an apparatus according to any of Claims 1 to 7 and comprises the steps of:
- providing a fresh culture medium storage vessel (3) containing fresh culture medium such that the inlet of the supply conduit (9, 10) is immersed within the culture medium;
- providing a cell culture vessel (5) containing culture medium such that the inlet of the drainage conduit (12) is immersed within the culture medium; and
- actuating the pump (2) to pump fresh culture medium from the fresh culture medium storage vessel (3) to the cell culture vessel (5) and withdraw used cell culture medium from the cell culture vessel to the used culture medium storage vessel (4).

9. A method as claimed in Claim 8 including a step of adjusting the rate of perfusion of cells with fresh culture medium by adjusting the pump remotely by means of wireless communication.

10. A method as claimed in Claim 8 where the fresh culture medium is a liquid suitable for culturing live cells.

11. A method as claimed in Claim 8 where fresh culture medium contains a drug substance and the apparatus is used for monitoring cell status over time.

12. A method as claimed in Claim 8 where the fresh culture medium contains substances required for promoting cell growth and division.

13. A method as claimed in Claim 8 where the fresh culture medium contains a substance capable of inducing cell differentiation between different cell types.

14. A method as claimed in Claim 8 where the fresh culture medium also contains a substance capable of inducing cells to produce cytokines, proteins, antibodies or other cell life cycle by-products.

15. A method of performing a toxicity assay on live cells, which method employs a method of perfusing live cells with cell culture medium according to Claim 8, wherein the cell culture medium includes a test agent, and wherein the method includes a step of assaying the toxicity of the test agent to the cells in the cell culture vessel.

## Patentansprüche

1. Vorrichtung (1) zur Ermöglichung des Austausches von Kulturmedium für lebende Zellen:
- ein Zellkulturbehälter (5);
- ein Vorratsbehälter (3) für frisches Kulturmedium;
- ein Sammelbehälter (4) für verbrauchtes Kulturmedium;
- eine Zuführungsleitung (9, 10), die zur Bereitstellung von Flüssigkeitsverbindung zwischen dem Vorratsbehälter (3) für frisches Kulturmedium und dem Zellkulturbehälter (5) geeignet ist;
- eine Abflussleitung (12), die zur Bereitstellung von Flüssigkeitsverbindung zwischen dem Zellkulturbehälter (5) und dem Sammelbehälter (4) für verbrauchtes Kulturmedium geeignet ist, wobei ein Einlass der Abflussleitung innerhalb des Zellkulturbehälters (5) angeordnet ist, sodass er sich bei Verwendung in dem Kulturmedium befindet; und
- eine Pumpe (2), die zum Pumpen von frischem Kulturmedium aus dem Vorratsbehälter (3) für frisches Kulturmedium zu dem Zellkulturbehälter (5) und zum Pumpen von verbrauchtem Kulturmedium aus dem Zellkulturbehälter (5) zu dem Sammelbehälter (4) für verbrauchtes Kulturmedium geeignet ist,
wobei:
der Zellkulturbehälter (5) nicht zur Umgebung abgeschlossen ist und die Vorrichtung eine Druckausgleichsleitung (11) einschließt, die zur Bereitstellung von Druckausgleich zwischen dem Sammelbehälter (4) für verbrauchtes Kulturmedium und dem Vorratsbehälter (3) für frisches Kulturmedium geeignet ist, und in der der Vorratsbehälter (3) für frisches Kulturmedium und Sammelbehälter (4) für verbrauchtes Kulturmedium zur Umgebung abgeschlossen sind.

2. Vorrichtung nach Anspruch 1, in der der Vorratsbehälter (3) für frisches Kulturmedium und der Sammelbehälter (4) für verbrauchtes Kulturmedium derart abgeschlossen sind, dass sie nicht mit der Umgebung verbunden sind, wobei nur eine Verbindung zu und von den Behältern (3) und (4) über die Zuführungsleitung (9, 10), Abflussleitung (12) und Ausgleichsleitung (10) vorliegt.

3. Vorrichtung nach Anspruch 1 oder 2, in der die Pumpe (2) in Reihe mit der Zuführung- oder Abflussleitung (9, 10, 12) bereitgestellt ist.

4. Vorrichtung nach einem der Ansprüche 1 bis 3, in der der Vorratsbehälter (3) für frisches Kulturmedium und der Sammelbehälter (4) für verbrauchtes Kulturmedium durch getrennte Kammern des einen Behälters (40) ausgebildet sind.

5. Vorrichtung nach einem der Ansprüche 1 bis 4, in der der Zellkulturbehälter aus einer Petrischale und einer Zellkulturflasche ausgewählt ist.

6. Vorrichtung nach einem vorstehenden Anspruch, in der die Pumpe zur Fernbedienung mithilfe eines drahtlosen Kommunikationssystems geeignet ist.

7. Vorrichtung nach einem vorstehenden Anspruch, in der der Zellkulturbehälter (5) zu der Umgebung nicht abgeschlossen ist.

8. Verfahren zum Perfundieren von Zellen in einem Zellkulturbehälter mit frischem Zellkulturmedium, welches Verfahren eine Vorrichtung nach einem der Ansprüche 1 bis 7 einsetzt und die folgenden Schritte umfasst:
- Bereitstellen eines Vorratsbehälters (3) für frisches Kulturmedium, der frisches Kulturmedium enthält, sodass der Einlass der Zuführungsleitung (9, 10) in dem Kulturmedium eingetaucht ist;
- Bereitstellen eines Zellkulturbehälters (5), der Kulturmedium enthält, sodass der Einlass der Abflussleitung (12) in dem Kulturmedium eingetaucht ist; und
- Betätigen der Pumpe (2), um frisches Kulturmedium aus dem Vorratsbehälter (3) für frisches Kulturmedium zu dem Zellkulturbehälter (5) zu pumpen und verbrauchtes Zellkulturmedium aus dem Zellkulturbehälter zu dem Vorratsbehälter (4) für verbrauchtes Kulturmedium abzuziehen.

9. Verfahren nach Anspruch 8, das einen Schritt des Einstellens der Rate der Perfusion von Zellen mit frischem Kulturmedium durch fernbedientes Einstellen der Pumpe mithilfe von drahtloser Kommunikation einschließt.

10. Verfahren nach Anspruch 8, wobei das frische Kulturmedium eine Flüssigkeit ist, die zur Kultivierung von lebenden Zellen geeignet ist.

11. Verfahren nach Anspruch 8, wobei frisches Kulturmedium eine Wirkstoffsubstanz enthält und die Vorrichtung zur Überwachung des Zellenstatus im Zeitverlauf verwendet wird.

12. Verfahren nach Anspruch 8, wobei das frische Kulturmedium Substanzen enthält, die für die Förderung von Zellwachstum und -teilung erforderlich sind.

13. Verfahren nach Anspruch 8, wobei das frische Kulturmedium eine Substanz enthält, die Zelldifferenzierung zwischen verschiedenen Zelltypen induzieren kann.

14. Verfahren nach Anspruch 8, wobei das frische Kulturmedium auch eine Substanz enthält, die Zellen zur Produktion von Cytokinen, Proteinen, Antikörpern oder anderen Nebenprodukten des Zelllebenszyklus induzieren kann.

15. Verfahren zur Durchführung einer Toxizitätsprüfung mit lebenden Zellen, welches Verfahren ein Verfahren zum Perfundieren von lebenden Zellen mit Zellkulturmedium nach Anspruch 8 einsetzt, wobei das Zellkulturmedium ein Testmittel einschließt und wobei das Verfahren einen Schritt des Prüfens der Toxizität des Testmittels für die Zellen in dem Zellkulturbehälter einschließt.

## Revendications

1. Appareil (1) pour permettre le remplacement d'un milieu de culture pour cellules vivantes :
- un récipient de culture cellulaire (5) ;
- un récipient de stockage de milieu de culture frais (3) ;
- un récipient de collecte de milieu de culture usagé (4) ;
- un conduit d'alimentation (9, 10) adapté pour fournir une communication fluidique entre le récipient de stockage de milieu de culture frais (3) et le récipient de culture cellulaire (5) ;
- un conduit de drainage (12) adapté pour fournir une communication fluidique entre le récipient de culture cellulaire (5) et le récipient de collecte de milieu de culture usagé (4), une entrée du conduit de drainage étant disposée au sein du récipient de culture cellulaire (5) de telle sorte que, lors de l'utilisation, il est situé dans le milieu de culture ; et
- une pompe (2) adaptée pour pomper du milieu de culture frais du récipient de stockage de milieu de culture frais (3) vers le récipient de culture cellulaire (5) et pour pomper du milieu de culture usagé du récipient de culture cellulaire (5) vers le récipient de collecte de milieu de culture usagé (4),
dans lequel :
le récipient de culture cellulaire (5) n'est pas scellé vis-à-vis de l'air ambiant, et l'appareil inclut un conduit d'égalisation de pression (11) adapté pour fournir une égalisation de pression entre le récipient de collecte de milieu de culture usagé (4) et le récipient de stockage de milieu de culture frais (3) et dans lequel le récipient de stockage de milieu de culture frais (3) et le récipient de collecte de milieu de culture usagé (4) sont scellés vis-à-vis de l'air ambiant.

2. Appareil selon la revendication 1 dans lequel le récipient de stockage de milieu de culture frais (3) et le récipient de collecte de milieu de culture usagé (4) sont scellés de telle sorte qu'ils ne peuvent pas communiquer avec l'air ambiant, la seule communication vers et depuis lesdits récipients (3) et (4) se faisant par l'intermédiaire du conduit d'alimentation (9, 10), du conduit de drainage (12), et du conduit d'égalisation (10).

3. Appareil selon la revendication 1 ou 2 dans lequel la pompe (2) est prévue en ligne dans le conduit d'alimentation ou de drainage (9, 10, 12).

4. Appareil selon l'une quelconque des revendications 1 à 3 dans lequel le récipient de stockage de milieu de culture frais (3) et le récipient de collecte de milieu de culture usagé (4) sont formés par des compartiments séparés dudit contenant (40).

5. Appareil selon l'une quelconque des revendications 1 à 4 dans lequel le récipient de culture cellulaire est choisi parmi une boîte de Pétri et un flacon de culture cellulaire.

6. Appareil selon l'une quelconque des revendications précédentes dans lequel la pompe est adaptée pour un fonctionnement à distance au moyen d'un système de communication sans fil.

7. Appareil selon l'une quelconque des revendications précédentes dans lequel le récipient de culture cellulaire (5) n'est pas scellé vis-à-vis de l'air ambiant.

8. Procédé de perfusion de cellules dans un récipient de culture cellulaire avec un milieu de culture cellulaire frais, lequel procédé emploie un appareil selon l'une quelconque des revendications 1 à 7 et comprend les étapes consistant à :
- fournir un récipient de stockage de milieu de culture frais (3) contenant un milieu de culture frais de telle sorte que l'entrée du conduit d'alimentation (9, 10) est immergée au sein du milieu de culture ;
- fournir un récipient de culture cellulaire (5) contenant un milieu de culture de telle sorte que l'entrée du conduit de drainage (12) est immergée au sein du milieu de culture ; et
- actionner la pompe (2) pour pomper du milieu de culture frais du récipient de stockage de milieu de culture frais (3) vers le récipient de culture cellulaire (5) et soutirer du milieu de culture cellulaire usagé du récipient de culture cellulaire vers le récipient de stockage de milieu de culture usagé (4).

9. Procédé selon la revendication 8 incluant une étape consistant à régler la vitesse de perfusion des cellules avec un milieu de culture frais par réglage à distance de la pompe au moyen d'une communication sans fil.

10. Procédé selon la revendication 8 où le milieu de culture frais est un liquide approprié pour une culture de cellules vivantes.

11. Procédé selon la revendication 8 où le milieu de culture frais contient une substance médicamenteuse et l'appareil est utilisé pour surveiller l'état des cellules au cours du temps.

12. Procédé selon la revendication 8 où le milieu de culture frais contient des substances nécessaires pour favoriser la croissance cellulaire et la division cellulaire.

13. Procédé selon la revendication 8 où le milieu de culture frais contient une substance capable d'induire une différenciation cellulaire entre différents types de cellules.

14. Procédé selon la revendication 8 où le milieu de culture frais contient également une substance capable d'induire les cellules à produire des cytokines, des protéines, des anticorps ou d'autres sous-produits du cycle de vie cellulaire.

15. Procédé de réalisation d'un essai de toxicité sur des cellules vivantes, lequel procédé emploie un procédé de perfusion de cellules vivantes avec un milieu de culture cellulaire selon la revendication 8, dans lequel le milieu de culture cellulaire inclut un agent de test, et dans lequel le procédé inclut une étape d'essai de la toxicité de l'agent de test vis-à-vis des cellules dans le récipient de culture cellulaire.
